# EUROPEAN PATENT APPLICATION

(11) **EP 4 385 401 A1**
(43) Date of publication of application: **19.06.2024**
(21) Application number: 22212996.7
(22) Date of filing: 13.12.2022
(51) Int. Cl.: A61B 5/0215, A61B 5/1495, A61B 5/157, G01N 33/96, A61B 5/145

(54) **SYSTEM FOR DELIVERING A SENSING DEVICE INTO A BODY**

(71) Applicant: Koninklijke Philips N.V., 5656 AG Eindhoven (NL)
(72) Inventor: GLEICH, Bernhard, Eindhoven (NL); RAHMER, Juergen Erwin, Eindhoven (NL)
(74) Representative: Philips Intellectual Property & Standards

(57) **Abstract**

The invention relates to a system (100) for delivering a sensing device (10; 20) into a body that allows to measure at least one property of a body fluid. The system comprises a storage portion (101) for storing the sensing device before delivery, the system allowing to withdraw body fluid from the body into the storage portion, and a reference sensor (102) configured to carry out a reference measurement of the property to be measured, the reference measurement being carried out on body fluid withdrawn from the body into the storage portion. After the reference measurement has been carried out, the sensing device can be delivered from the storage portion into the body. The presented system allows to simplify accurate measurements of body fluids.

## Description

### FIELD OF THE INVENTION

The invention relates to a system for delivering a sensing device into a body, to a calibration apparatus for calibrating the sensing device, a calibration method for calibrating the sensing device, a method for using the system, and to a computer program for calibrating the sensing device.

### BACKGROUND OF THE INVENTION

In order to measure some properties of body fluids, it may be necessary that a corresponding sensing device is located inside the fluid. For instance, pressure measurements using pressure sensors located inside the fluid whose pressure is to be measured typically yield more accurate results than others. Moreover, it may be necessary in clinical practice that the property of the body fluid is measured inside the body, which means that the sensing device also may need to be inserted into the body, as would be the case, for instance, for invasive blood pressure measurements. Apart from other risks that might be associated with an insertion of sensing devices into a body, this also raises problems regarding measurement accuracy. This is because the insertion of the sensing device goes along with a change in an environment for the sensing device, wherein this change may affect a sensing behavior of the sensing device, thereby possibly decreasing measurement accuracy. One may attempt to address this problem by calibrating the sensing device after insertion, i.e. while it is already inside the body. However, it would be desirable if a sensing device could be calibrated beforehand, since this can simplify the calibration, the latter being necessary for accurate measurements. Moreover, in the clinical context, medical risks can be decreased when not having to calibrate the sensing device *in situ.*

### SUMMARY OF THE INVENTION

It is an object of the invention to simplify accurate measurements of properties of body fluids.

In a first aspect of the invention, a system for delivering a sensing device into a body has been found, wherein the sensing device allows to measure at least one property of a body fluid inside the body, wherein the system comprises:
- a storage portion for storing the sensing device before delivery, wherein the system allows to withdraw body fluid from the body into the storage portion,
- a reference sensor configured to carry out a reference measurement of the property to be measured by the sensing device, wherein the reference measurement is carried out on body fluid withdrawn from the body into the storage portion, wherein the system allows to deliver the sensing device from the storage portion into the body after the reference measurement has been carried out.

When using the still stored sensing device to also carry out a measurement for the withdrawn body fluid, this measurement and the reference measurement can be used to calibrate the sensing device, i.e. in preparation of the measurements carried out by the sensing device after delivery into the body. This allows for a simpler calibration, which can particularly be medically favorable. Hence, accurate measurements of properties of body fluids can be carried out in a simpler way.

The system has been found in the context of blood pressure measurements using micro-magnetic resonators as disclosed, for instance, in WO 2020/253977 A1, which is herewith incorporated in its entirety by reference. However, the system could just as well be used for delivering other sensing devices, which may be used for measuring other properties of body fluids, including properties of other body fluids than blood. Still, since micro-magnetic resonators have been found to be particularly sensitive to changes in environmental conditions, particularly temperature changes occurring upon injection of the micro-magnetic resonators into the body, the system can be expected to be particularly useful for measurements using micro-magnetic resonators as sensing devices.

The body fluid for which a property is to be measured is preferably blood. Moreover, the property to be measured by the sensing device is preferably a blood pressure. Pressure parameters of particular medical importance include pulmonary (i.e., lung) artery pressure and wedge pressure. These pressures, which are preferred as properties to be measured by the sensing device to be delivered, have been found to be particularly useful in evaluating a patient's cardiac function. Usually, the pulmonary artery pressure as well as the wedge pressure are, however, measured using a catheter. Catheter procedures are costly, inconvenient for the patient and usually require sedation. Micro-magnetic resonators, which could also be referred to as magneto-mechanical oscillators (MMO), provide a technology for measuring blood pressure. In fact, they are suitable for measuring pulmonary artery pressure and wedge pressure. However, injecting an MMO into a patient, as indicated above, exposes the MMO to a blood environment, wherein a corresponding change in temperature will likely cause a change in the pressure sensed by the MMO. While an *in situ* calibration of the MMO using a catheter could circumvent this problem, the system presented herein allows for a considerably simpler calibration, overcoming the need for a catheter procedure when sensing, for instance, pulmonary artery pressure or wedge pressure using MMOs.

The sensing device to be delivered may be suitable for measuring more than one property, i.e. a plurality of properties, of a body fluid inside a body. For doing so, the sensing device can preferably be fully inserted into the body and float and/or flow in the respective body fluid. The sensing device could therefore also be regarded as an autonomous device, or a "bot". The sensing device has preferably a size in the micrometer range or even below.

The one or more properties measurable by the sensing device are not necessarily blood pressure parameters like the pulmonary artery pressure or wedge pressure, or properties indicative thereof, but may also be other blood properties and/or properties of other body fluids than blood. Other blood properties include other hemodynamic parameters or parameters indicative of a blood composition. Other body fluids include extracellular fluids, for instance. Apart from properties of fluids inside a patient's body, also properties of fluids inside more general object bodies could be measured. In other words, the presented system could, in principle, also be used for delivering sensing devices into object bodies for measurements in other than medical contexts. Irrespective of the particular fluid, pressure and temperature may be among the properties of interest and therefore measureable by the respective sensing device.

It will be understood that the presented system in itself may, unlike its intended use, not or hardly depend on the one or more properties measurable with the sensing device to be delivered with it, including which body fluid they relate to. Structurally, the system may in fact mainly or even solely depend on the sensing device to be delivered insofar as the sensing device needs to be storable and deliverable by the system. While, in principle, more involved implications by the type of the sensing device on the presented system for delivery are possible, in many cases the system may depend on the sensing device mainly or solely through the outer size and shape of the sensing device, and possibly its weights and/or its floating properties, but not its particular sensing mechanism.

The system can, particularly in the case of a blood pressure measurement, be used to deliver the sensing device into a vein, such as a peripheral vein, for instance. In case the property to be measured is a pulmonary artery pressure, for instance, the sensing device, which may then preferably be chosen to be a micro-device like an MMO, may travel from the vein to the lung.

Substantial changes in sensing characteristics, i.e. characteristics of the respective sensing device, have been observed to be correlated with temperature changes, and specifically changes in temperature of the body fluid whose property is to be measured. The temperature can itself, i.e. directly, change the sensing characteristics, or do so indirectly. For instance, for small pressure sensors like specifically MMOs, a pressure value determined by use of the pressure sensor may be affected by, i.e. depend on, a surface tension between the fluid whose property is to be measured and the sensing device, wherein the surface tension will typically be temperature-dependent.

A main effect on the sensing characteristics may still be due to a physical contact of the respective sensing device with fluid. This may also be referred to as "wetting". For instance, the temperature dependency of the above-mentioned surface tension may be less relevant compared to the fact that the surface tension between the fluid and the sensing device builds up at all. Moreover, the physical contact of the sensing device to the fluid may change also other properties of the sensing device potentially closely related to sensing characteristics, such as material properties. For instance, properties of an outermost coating of the sensing device related to sensing characteristics may depend on whether the sensing device is in physical contact with fluid, and particularly which kind of fluid. For example, water of a surrounding fluid can be incorporated in polymeric layers of a sensor device's coating. While such further effects can already be accounted for by calibrating the sensing device in the particular fluid whose property is to be measured, the calibration accuracy can be further increased by also taking into account temperature effects. Therefore, in an embodiment, the system can further comprise a temperature control unit configured to control a temperature of the body fluid withdrawn from the body into the storage portion. This allows for a particularly accurate calibration of the sensing device, since the sensing device cannot only be calibrated in the right fluid, but also at the right temperature. The temperature control unit can, in particular, be configured to maintain the withdrawn fluid at body temperature. Additionally or alternatively, the temperature control unit can be configured to adjust a temperature of the withdrawn fluid. It can comprise, for instance, a temperature sensor configured to measure a temperature of the withdrawn body fluid and a heater configured to heat the withdrawn body fluid based on the temperature measured by the temperature sensor.

Moreover, the system can comprise a further temperature sensor configured to measure a temperature inside the body. This further temperature sensor can be positioned at a distal end of the system. In this way, when, for instance, injecting the sensing device into a patient's vein, the further temperature sensor can be located in the patient's blood. The further temperature sensor therefore allows to measure the blood temperature more exactly, namely as it is directly in the blood stream. Since patients may differ in temperature, a set point of the temperature of the withdrawn blood in the system, as regulatable by the temperature control unit, may thus be changed to reflect an individual body temperature. Instead of using the further temperature sensor, it would also be possible to carry out a body core temperature measurement of the patient before, preferably directly before, delivering the sensing device, and to transfer the measured temperature into the system, particularly the temperature control unit, either manually or automatically. For instance, a corresponding data link between a device used for the body core temperature measurement before sensing device delivery and control electronics of the system may be used for this purpose.

The temperature control unit is preferably positioned proximal with respect to the further temperature sensor. It is preferably part of the storage portion. Thus, the storage portion can comprise a temperature control portion comprising the temperature control unit. Already this can allow for an efficient and accurate temperature control over the withdrawn body fluid and hence the stored sensing device. In fact, the storage portion may be constructed so as to store the sensing device in the temperature control portion. Hence, a storage position of the sensing device in the system may be in the temperature control portion, which comprises the temperature control unit. In this way, a specifically efficient and accurate temperature control over withdrawn body fluid in a vicinity of the stored sensing device and hence the stored sensing device itself can be achieved. The temperature control unit can be configured to heat the withdrawn body fluid in the temperature control portion of the system, possibly so as to maintain a temperature of the body fluid at body temperature. Since the sensing device gets in contact with the withdrawn and heated body fluid, heating the body fluid allows to heat the sensing device. In an embodiment, the stored sensing device could thus be said to be brought to body temperature.

The system may, furthermore, comprise an injection portion to be inserted into a delivery region inside the body, wherein body fluid can be withdrawn from the delivery region through the injection portion into the storage portion, and the sensing device can be injected from the storage portion through the injection portion into the delivery region inside the body. Hence, the withdrawal of body fluid and the injection of the sensing device can be achieved through one and the same portion of the system, which makes the system structurally less complex and allows for a minimally invasive delivery of the sensing device.

As already indicated, the delivery region may particularly be a patient's vein. The injection portion may be formed by a needle, particularly a distal part of a needle, i.e. it may have a hollow cylindrical shape whose distal end is pointed or sharp enough to break through a skin barrier upon reasonable application of pressure, and whose proximal end allows fluid like blood from the delivery region inside the body to enter the storage portion of the system. Instead of a needle-based injection mechanism, the fluid conducting relationship between the delivery region and the storage portion may, however, also be established differently. For instance, particularly if the body fluid of concern is not blood, whose access requires typically breaking of skin barriers, the injection portion may also correspond to a tube, wherein the tube may be inserted through already generated or even natural openings of a patient's body to reach the delivery region.

It may be preferred that the storage portion comprises a reference measurement portion comprising the reference sensor. Thus, the system can comprise a) a storage portion comprising i) the reference measurement portion and ii) the temperature control portion, and b) the injection portion. The storage portion, which will itself be located proximal with respect to the injection portion, may comprise the reference measurement portion at its proximal side and the temperature control portion at its distal side. In such a configuration, thus, the temperature control portion could be located, as seen in delivery direction, between the reference measurement portion and the injection portion. Preferably, for instance, the temperature control portion and the injection portion are both formed by a needle as mentioned above, wherein then the temperature control portion could correspond to a proximal side of the needle, and the injection portion could correspond to a distal side of the needle.

The reference measurement portion can have a larger extent than the injection portion in a transverse direction with respect to a delivery direction. In this way, convection in the body fluid withdrawn from the body into the storage portion can be increased, which may lead to a faster thermal equilibration, i.e. shorter acclimatizing times of the sensing device stored in the storage portion to the withdrawn body fluid. For instance, the reference measurement portion, which will typically be arranged coaxially and proximal with respect to the injection portion and possibly also the temperature control portion, may also be of a hollow, cylindrical shape. A diameter of the cylinder defining the shape of the reference measurement portion may then be larger than a diameter of the cylinder defining the shape of the injection portion and possibly the temperature control portion. The diameter may, in each case, be measured transverse to, particularly perpendicular to, the delivery direction. The delivery direction refers to a direction along which the sensing device is delivered from the storage portion into the body. Hence, for instance, if the reference measurement portion and a part of the system jointly formed by the temperature control portion and the injection portion are both cylindrically shaped and coaxial with respect to each other, then the delivery direction may refer to a direction along the common axis pointing towards the body for delivery of the sensing device. It is understood that the reference measurement portion having a larger extent than the injection portion, and possibly the temperature control portion, in the transverse direction with respect to the delivery direction may also be viewed as the injection portion, and possibly the temperature control portion, having a smaller extent in the transverse direction with respect to the delivery direction. This can allow for an easier delivery of the sensing device.

As indicated above, it may be beneficial that the storage portion comprises the reference sensor, namely in the reference measurement portion. Hence, for instance, the reference sensor can be positioned at, in or on the reference measurement portion of the system. In this way, the reference measurement can be carried out on the withdrawn body fluid having entered the storage portion in a relatively simple manner. Similarly, the temperature control unit can particularly be positioned at, in or on the temperature control portion. For instance, the temperature control unit can in this way be positioned, as viewed in the delivery direction, between the reference sensor of the reference measurement portion and a further reference, or calibration, sensor at a distal end of the injection portion, the latter possibly corresponding to the further temperature sensor mentioned above. If the sensing device is not stored in the temperature control portion, but instead more proximal, the body fluid may hence be preconditioned, particularly preheated, before getting in contact with the sensing device. Also this may reduce an acclimatizing time for the sensing device. A heating of the fluid may be achieved more efficiently along the temperature control portion as compared to, for instance, the reference measurement portion, if the temperature control portion is narrower than the reference measurement portion.

For withdrawing the body fluid from the body into the storage portion, the system may comprise a pressure generator for generating underpressure inside the system relative to the delivery region if the injection portion is inserted with its distal part into the delivery region. This allows for an efficient withdrawal of body fluid from the body. The pressure generator may further allow to generate a mechanical force on the sensing device for delivering the sensing device from the storage portion into the body. In this way, the sensing device can be safely delivered into the body. Optionally, both the withdrawal of the body fluid and the delivery of the sensing device into the body, possibly together with at least a part of the previously withdrawn body fluid, can be achieved based on pressure generation. However, this could be regarded as an alternative, since also only the former may be the case, i.e. even if the body fluid is withdrawn by generating underpressure, the sensing device may not necessarily be delivered into the body by overpressure, but may instead be, for instance, pushed into the body. It is understood that such a pushing action may also be accompanied by some overpressure generation inside the system. In fact, also underpressure generation may not be needed, since the pressure of the body fluid may be sufficient for it to be withdrawn into the storage portion.

The generation of pressure may be achieved, for instance, using a piston arrangement. However, in other embodiments, pressure can be generated in different ways as well, such as by a pump. In fact, mechanical force generation may be achieved by a piston as well.

In an example, the system comprises a single piston arrangement both for withdrawing body fluid from the body into the storage portion and for delivering the sensing device from the storage portion into the body. The piston arrangement can comprise a piston whose position can determine a size of the storage portion, wherein the size may refer to a volume fillable by body fluid. For instance, from a first position, in which the piston is positioned such that the storage portion is relatively small in volume, the piston may be pulled into a second position in which the storage portion may be relatively large in volume. Pulling the piston from the first to the second position may or may not generate an underpressure, but irrespective of this cause the body fluid to be withdrawn from the body into the simultaneously growing storage portion of the system. In principle, the piston may afterwards be pushed back from the second position into the first position, and thereby deliver the sensing device, together with the previously withdrawn body fluid, into the body.

Instead of delivering the sensing device together with the previously withdrawn body fluid into the body by pushing the piston as a whole, the piston arrangement may preferably comprise a further piston, which may be part of the previously mentioned piston in the form of an independently movable sub-piston, wherein this further piston may be used to push the sensing device into the body substantially without decreasing the volume of the storage portion, thereby substantially without delivering any previously withdrawn body fluid back into the body. Hence, the piston arrangement may comprise a first piston for generating underpressure in order to withdraw the body fluid from the body, and a second piston in the form of a sub-piston of the first piston for pushing the sensing device into the body. The sub-piston may be a part of the first, "main" piston and movable with respect to it, possibly arranged on an axis of the first piston. It should be noted that also a combination of pressure and a mechanical action may be used for delivering the sensing device into the body. For example, after moving the first piston from the first position into the second position, thereby withdrawing fluid from the body into the storage portion in which one or more sensing devices are stored, the first piston may, together with the second piston, be moved back from the second position into the first position (or, in fact, only a position in between the second position and the first position), thereby pressing part of the previously withdrawn body fluid back into the body, whereafter a second piston may be used to push any sensing device that has not yet flown along with the pressed-back part of the body fluid into the body further, thereby finally causing it to reach the interior of the body. In embodiments where a storage position of the sensing device is in a part of the storage portion that is relatively narrow, for instance, the first piston may not fit into that part and additionally the injection portion in order to reach and deliver the stored sensing device, but the second piston may, such that only the second piston may be used to push the sensing device and thereby finally deliver it into the body.

Geometrically, the system may, in an embodiment, extend along a longitudinal axis corresponding to the delivery direction, wherein the storage portion forms a first, proximal portion of the system in the delivery direction, and the injection portion forms a second, distal portion of the system in the delivery direction, wherein the temperature control portion is a distal section of the storage portion preceding the injection portion in the delivery direction and having the same extent as the injection portion in the transverse direction with respect to the delivery direction, and wherein the reference measurement portion is a section of the storage portion preceding the temperature control portion in the delivery direction. This geometry has been found to be particularly useful in practice.

As already mentioned, the delivery direction refers to a direction along which the sensing device is delivered from the storage portion into the body. Correspondingly, when referring to parts of the system for delivering a sensing device, the terms "proximal" and "distal" refer to positions along the delivery direction, wherein a "proximal" position precedes a "distal" position in the delivery direction and *vice versa.* For instance, in a state in which a sensing device is in the course of being injected into a body by use of the system, distal parts of the system would be closer to the body than proximal parts of the system and *vice versa.*

The temperature control portion preferably forms, together with the injection portion, a uniform part of the system. In particular, the storage portion up to the temperature control portion may form a first, proximal hollow cylinder, and the temperature control portion may, together with the injection portion, form a second, distal hollow cylinder, wherein the first hollow cylinder has a larger extent in the transverse direction with respect to the delivery direction than the second hollow cylinder. A transition between the first and the second hollow cylinder may be formed by, i.e. between, the reference measurement portion and the temperature control portion. Fluid can flow between the first and the second hollow cylinder via the transition. As stated above, the sensing device may be stored in the temperature control portion.

The storage portion is herein defined so as to comprise both the reference measurement region and the temperature control portion, and therefore particularly to extend beyond where the extent of the system in the transverse direction with respect to the delivery direction changes. However, the storage portion could also be defined not to include the temperature control portion, and therefore particularly to not extend beyond where the extent of the system in the transverse direction with respect to the delivery direction changes. Instead, the temperature control portion could be defined as being included in the injection portion. Then, for instance, the storage portion could correspond to the first, proximal hollow cylinder and the injection portion could correspond to the second, distal hollow cylinder. As outlined above, the presented system allows for a calibration of the sensing device before its delivery into the body, thereby simplifying accurate measurements of properties of body fluids. Correspondingly, in an aspect, the invention also relates to a calibration apparatus for calibrating a sensing device allowing to measure a property of a body fluid inside the body and to be delivered into the body by the system described above. Hence, the calibration apparatus cooperates with, or can be used in cooperation with, the above system for delivering the sensing device, to realize simplified accurate measurements of properties of body fluids. The calibration apparatus comprises:
- a calibration signal providing unit configured to provide a calibration signal, the calibration signal being a signal acquired from the sensing device with the sensing device being stored in the storage portion of the system after withdrawal of body fluid into the storage portion,
- a reference measurement data providing unit configured to provide reference measurement data acquired using the reference sensor, and
- a calibration unit configured to calibrate the sensing device based on a) the calibration signal and b) the reference measurement data. In particular, the reference measurement data may be acquired contemporaneously with the calibration signal.

It is understood that a calibration of the sensing device does not necessarily change the sensing device itself in any way. Instead, calibrating the sensing device will typically refer to learning how to interpret signals acquired from the sensing device. The calibration signal is preferably a signal as it would also be used for measurement once the sensing device has been delivered into the body, meaning that the calibration signal is preferably acquired just like a measurement signal would be acquired from the sensing device, with the exception that the sensing device is still stored in the storage portion of the system.

The reference sensor is preferably trusted. For instance, it may be pre-calibrated, wherein its accuracy will set an upper bound for any measurement of the sensing device. The reference measurement data indicate a certain value of the property to be measured, or values of this property over time. The calibration unit can therefore be configured to calibrate the sensing device by associating the calibration signal with the one or more values indicated by the reference measurement data. If, then, during measurement inside the body, signals are acquired from the sensing device corresponding to a certain calibration signal, the property to be measured can be assumed to have the respective value associated to the acquired signal during calibration also in the region in which the sensing device is located in the body.

A related aspect concerns a measurement system for measuring a property of a body fluid inside a body using a sensing device to be delivered into the body, wherein the measurement system comprises the system described above and the calibration apparatus, as also described above. Also one or more sensing devices could be considered as being part of the measurement system, wherein the one or more sensing devices may or may not already be stored in the system. The calibration unit can comprise, correspond to or be part of an evaluation unit used to evaluate a measurement carried out with the delivered sensing device, wherein this evaluation unit would preferably be part of the measurement system. The evaluation unit can be configured to determine the property to be measured for body fluid surrounding the delivered sensing device inside the body based on a) the calibration previously carried out using the reference sensor and the stored sensing device and b) a measurement carried out using the delivered sensing device.

The sensing device is preferably constructed and/or configured such that wireless signals can be acquired from it. Hence, the calibration signal and any measurement signals acquired from the sensing device are preferably wireless signals. In particular, the signals can be electromagnetic signals, as is the case for magneto-mechanical resonators. Typically, the sensing device would then be constructed and/or configured to be excitable by an external excitation field so as to emit response signals. For instance, the calibration apparatus and/or the measurement system can comprise a field generator for generating a magnetic or electromagnetic excitation field, a transducer for transducing a magnetic or electromagnetic field generated by the sensing device in response to being excited by the excitation field into electrical response signals and a processor for determining values of the property to be measured based on the electrical response signals. The processor can be or correspond to the evaluation unit mentioned above. In the case of magneto-mechanical resonators, the excitation field may induce mechanical oscillations of one or more magnetic objects in the resonator, wherein the induced mechanical oscillations of the one or more magnetic objects generate the field that is transduced into the electrical response signals.

The calibration unit may be configured to calibrate the sensing device based on a calibration signal and reference measurement data acquired over a calibration time and/or at least at a time at which the sensing device has thermally equilibrated with the withdrawn body fluid to a predetermined degree. In this way, the calibration quality can be improved, since it will typically take some time for the sensing device to reach a measurement region inside the body after delivery, such that measurement signals will likely be acquired from the sensing device in a state of thermal equilibrium, or at least approximate thermal equilibrium, with the body fluid surrounding it. It is understood that, for carrying out such a calibration, corresponding calibration signals and reference measurement data may be acquired beforehand. In particular, the calibration signal may be continuously acquired, and the reference measurement data continuously recorded between withdrawal of body fluid and delivery of the sensing device. Then, the calibration unit may be configured to recognize from the calibration signal and the reference measurement data how, particularly when, the sensing device thermally equilibrates to the withdrawn body fluid.

A calibration of the sensing device after it has adapted to surrounding body fluid at body temperature may be most important and therefore preferred. However, in principle, the sensing device may also be calibrated for changes in temperature. This may be beneficial in case the temperature of the body fluid inside the body, i.e. during the actual measurement that is to be carried out, is expected to change in the course of the measurement, and if the property to be measured is temperature-dependent. In order to calibrate the sensing device for a temperature dependence, for instance, the temperature control unit can be configured to control the temperature of the withdrawn body fluid such that it takes a predefined number of, or sweeps a predefined range of, values, wherein calibration signals and associated reference measurement data are provided for a plurality of different of the temperatures.

Moreover, the calibration unit may be configured to calibrate a collision behavior of the sensing device based on mechanical collisions between the sensing device and its surroundings detected in the calibration signal. For example, the calibration apparatus may be configured to calibrate the sensing device based on a calibration signal and reference measurement data acquired over a calibration time extending at least up to delivery, wherein the calibration unit may be configured to calibrate the sensing device based on calibration mechanical collisions between the sensing device and its surroundings up to delivery detected in the calibration signal, particularly in relation to the reference measurement data. In this way, effects of mechanical collisions on measurement signals, which are relatively likely to be experienced during measurement due to mechanical collisions of the sensing device inside the body also being relatively likely, can be estimated and therefore taken into account when interpreting the measurement signals. Hence, more accurate measurements of the property to be determined become possible.

In the case of magneto-mechanical resonators as sensing devices, abrupt changes, or "bumps", in the signal acquired from the device have been associated with situations where the sensing device is squeezed too much due to direct mechanical action of certain tissue structures of the body on it. Such tissue structures may be vessel walls, muscles in the vessel system, especially in the heart, heart valve flaps, *et cetera.* It is possible that these tissue structures squeeze the sensing device much harder than the blood pressure could, which can result in a permanent (plastic) or semi-permanent (visco-elastic) deformation of the sensor. Therefore, the acquired signal may be changed permanently or may drift for a while, depending on the kind of deformation. Whether the sensing device responds in a plastic or in a visco-elastic manner, or according to a combination of the two, is preferably determined before measurement, and therefore as part of the calibration. It may be favorable to carry out this determination on a different sensing device, i.e. not the sensing device actually to be used for measurement, wherein the different sensing device is of the same type as the sensing device to be used for measurement. Nevertheless, it could also be acceptable to carry out the determination of the deformation behavior on the sensing device to be used for measurement itself, particularly in case the deformations to be expected are mainly in the visco-elastic regime.

The calibration unit, just like the evaluation unit, may be configured to detect mechanical collisions of the sensing device based on sudden changes in the signal acquired from the device. A signal change may be considered "sudden" in case the change is higher than a predetermined threshold in a predetermined amount of time. How the signal changes in detail, particularly whether the signal returns to its previous value, can be used by the calibration unit to characterize the deformation behavior, which could also be regarded as a collision behavior, of the sensing device. It is reasonable to assume that the value of the property to be measured does not change in the course of the collision. Hence, a signal change that persists, i.e. a signal shift from before to after the collision, can be taken as an indicator for an at least partially plastic deformation behavior of the sensing device. During measurement, hence, a corresponding shift, i.e. backshift, of the signal can be applied after having detected a collision. In the case of a visco-elastic behavior or a mixed type behavior of the sensing device, a model of the response of the sensing device to mechanical collisions can be determined by the calibration unit during calibration upon detecting mechanical collisions, wherein a correction of subsequent measurement signals can be applied based on this model. Still, also in the case of visco-elastic or mixed type behavior, it is reasonable to assume that no actual jump in the property to be measured happens in mechanical collisions.

The signal considered for detecting mechanical collisions can be a corrected version of an actual signal acquired from the sensing device, wherein the correction can comprise a correction for known variations in the signal, particularly variations that could be mistaken as indicating mechanical collisions. For instance, if the sensing device is a pressure sensor for measuring a blood pressure, pressure variations due to the heart beat can be eliminated from the signal. In other words, the blood pressure may be measured relative to the heart beat, such that the signal considered for detecting mechanical collisions could already be corrected for the heartbeat. During calibration, a lack of change in the pressure to be measured due to mechanical collisions may hence be assumed relative to the heart beat.

Apart from being suitable for measuring the body fluid property, the sensing device is preferably locatable. In case of being a micro-magnetic resonator, for instance, it may be locatable due to its unique response properties to received electromagnetic radiation. These unique response properties may be characterized, for instance, by a resonance frequency of the resonator. Hence, the calibration unit may be configured to also derive position data of the sensing device from the calibration signal, and to calibrate the sensing device based also on this position data. The position data collected, or determined, during calibration may have a preliminary accuracy, wherein position data of the sensing device having an improved accuracy may be acquired based on a result of the calibration.

Also hydrostatic effects may be accounted for during calibration. This can be beneficial, since it has been found that also hydrostatic effects can influence the measurement signal without actually affecting the property to be measured. For instance, if the property to be measured is a blood pressure, this pressure typically needs to be given relative to a well-defined position, which will often be the heart. This position can, for instance, be predefined to be a particular position in the heart, or just the center of the heart. When measuring pressure on a different height, i.e. at a different gravitational potential, the measured pressure will differ from the actually wanted pressure. However, this pressure difference can be approximated to a sufficient degree by assuming it is proportional to the height difference, i.e. the height difference between the height at which the sensing device is actually located during signal acquisition and the height of the position relative to which pressure shall be measured, i.e., for instance, the center of the heart. The constant of proportionality between the difference in measured and actually wanted pressure on the one hand, and the height difference on the other hand is the product of the density of the respective body fluid, i.e., particularly blood, and the (local) gravitational acceleration. Blood density is approximately similar in all patients, and the gravitational acceleration is also not considerably different around the globe. Therefore, it can be sufficient to measure the height of the sensing device over time. Additionally, at one time, the height of the sensing device relative to the reference point (e.g., the center of the heart) should be determined, assuming the patient does not move significantly. As the position of the sensing device relative to a transducer or any other measurement devices used for acquiring the signal of the sensing device, such as measuring coil arrays, for instance, can be measured, the orientation of the measurement devices with respect to the gravitational field is preferably also measured. This could, for instance, be done during setup, such as by means of levelling, or by a dedicated sensor in the measurement devices. The reference position, i.e., the center of the heart, for instance, can be introduced by placing the sensing device at the height of the heart, placing a different sensing device at this height or evaluating the track, i.e., the spatial path, of the sensing device and determining the heart position based on a characteristic movement shape and/or velocities of the sensing device.

Since the calibration can be carried out before delivery of the sensing device, such that the sensing device no longer needs to be calibrated inside the body, a duration over which the sensing device needs to be inside the body can be considerably shortened. Hence, the presented system and the above calibration apparatus allow for a use of sensing devices that dissolve in the body fluid, such as within minutes. In an aspect, therefore, the present invention also relates to a sensing device for delivery into a body by a system as described above, wherein the sensing device is dissolvable in the body fluid, particularly within minutes. For instance, the sensing device can comprise or consist of poly-lactic acid of a predetermined thickness. Short-term determinations of pulmonary artery pressure therefore become possible, for instance.

The system, i.e. the system for delivering the sensing device into a body, does not need to be dissolvable. But, it can be preferred that low frequency magnetic fields can penetrate the system easily, in order to not disturb signals acquired from the sensing devices. Therefore, low conductivity materials may be preferred for the system, especially polymers and ceramics. For some parts of the system, such as the distal part of the needle forming the injection portion in an embodiment, low-conductivity metals may be used, such as, for instance, titanium alloys, stainless steel, *et cetera.*

In case the property to be measured is a wedge pressure, the sensing device preferably comprises a spherical casing and two sensing sub-devices inside the casing, wherein each of the sub-devices is positioned in a respective chamber of the sensing device, the chambers allowing body fluid from outside to enter the respective chamber, but not from one chamber into the other.

Wedge pressure can be regarded as a proxy for pulmonary vein pressure. It can be measured by closing off a sufficient diameter vessel in the pulmonary arterial system and then measuring the downstream pressure. This pressure is usually close to the venous pressure, assuming there is no significant collateral arterial inflow behind the respective closing object, which could also be regarded as a plug. The sensing device to be delivered with the system can be regarded as a floating system. In order to carry out the wedge pressure measurement using this floating system, which should close the artery, the shape of the system that has been found to be preferred is a spherical shape. Alternatively, for instance, a prolate ellipsoid may be used, wherein an aspect ratio of the respective shape should however not exceed a predetermined threshold, such as it would be the case for very long and narrow cigars, for instance. Since the orientation of the sensing device can hardly be controlled, having at least two sensing sub-devices in the whole device is preferred, since in this way one of the sub-devices will point in the right direction. For a spherical device, more than two sub-devices can increase the chance that one of the sub-devices points in the right direction, since for spheres there is a risk that an inlet for the body fluid in one of the respective chambers housing the sub-devices is blocked when the whole device closes the vessel.

It will be understood that the invention also relates to a calibration method for calibrating a sensing device that allows to measure a property of a body fluid inside the body and to be delivered into the body by the system as defined above. The calibration method includes the following steps:
- providing a calibration signal, the calibration signal being a signal acquired from the sensing device with the sensing device being stored in the storage portion of the system after withdrawal of body fluid into the storage portion,
- providing reference measurement data acquired using the reference sensor, and
- calibrating the sensing device based on a) the calibration signal and b) the reference measurement data. The reference measurement data may particularly be acquired contemporaneously with the calibration signal.

In a further aspect, the invention relates to a method for using the system as described above. This method, which could also be regarded as a preparation method, includes the following steps:
- storing the sensing device in the storage portion,
- withdrawing body fluid from the body into the storage portion,
- carrying out, using the reference sensor, a reference measurement of the property to be measured by the sensing device, wherein the reference measurement is carried out on body fluid withdrawn by the system from the body into the storage portion,
- carrying out, using the stored sensing device, a calibration measurement of the property to be measured by the sensing device, wherein also the calibration measurement is carried out on the body fluid withdrawn by the system from the body into the storage portion,
- calibrating the sensing device based on the reference measurement and the calibration measurement, and
- delivering the sensing device from the storage portion into the body. The step of calibrating the sensing device based on the reference measurement and the calibration measurement can be carried out in accordance with the calibration method indicated above. In fact, already the above indicated calibration method could be viewed as a method of using, since by providing the calibration signal and the reference measurement data, information acquired with the sensing device and, respectively, the reference sensor of the system for delivering the sensing device into the body is used. Also both of the above methods therefore allow to realize simplified accurate measurements of properties of body fluids.

Storing the sensing device in the storage portion can be achieved, for instance, by clamping. For this purpose, the sensing device would preferably comprise clamps, and dimensions of the storing portion, particularly at the position at which the sensing device is actually to be stored in the storage portion, and the sensing device will typically be adapted to each other. For instance, a diameter of the storage portion at the respective storage position in a direction transverse to the delivery direction may be chosen so as to allow the sensing device to be clamped to inner walls of the storage portion at the storage position. MMOs often inherently comprise clamps. However, also other sensing devices can be provided with clamps, such as clamps like those known from MMOs. These clamps typically comprise three or more wires forming a sphere-like structure if not squeezed. When inserting a sensing device comprising clamps like these into part of a storage portion with a small enough dimension, such as a sufficiently narrow needle as mentioned above as a possible structure forming the temperature control portion and the injection portion, for instance, the clamps are squeezed and therefore can hold the respective sensing device in place. Using elements like wires for a clamp allows fluid to flow at the side of the sensing device (both inside the system and afterwards inside the body). For a wedge pressure sensor, for instance, a spring structure corresponding to the clamp is preferably part of the system, since the wedge pressure sensor is preferably strictly spherical, i.e. preferably comprises no elements like clamp wires itself. The clamp structure used as part of the system can, however, correspond to a clamp structure as it would be used as part of a sensing device. Hence, for instance, three wires can stick out at inner walls of the storage portion at the storage position and hold the sensing device in place while allowing fluid to flow around the sides of the sensing device, i.e. to pass by it. It may be preferred that the wires usable as part of the system to store sensing devices like wedge pressure sensors, which are preferably approximately spherical or ellipsoidal, form a cup-like configuration for accommodating such sensing devices.

A more general aspect of the invention concerns a use, i.e. any use, of the system as described above for delivering a sensing device into a body, and/or of the above described calibration apparatus.

Moreover, the invention relates to a computer program for calibrating a sensing device that allows to measure a property of a body fluid inside the body, to be delivered into the body using the system as described above, the program comprising instructions causing the calibration apparatus from above to carry out the above described calibration method. The calibration apparatus is therefore preferably a data processing apparatus, i.e. a computer. Also the computer program helps to realize simplified accurate measurements of properties of body fluids.

It shall be understood that the system of claim 1, the calibration apparatus of claim 10, the calibration method of claim 13, the method of claim 14, and the computer program of claim 15, have similar and/or identical preferred embodiments, in particular, as defined in the dependent claims.

It shall be understood that a preferred embodiment of the present invention can also be any combination of the dependent claims or above embodiments with the respective independent claim. It will be understood from the above that, while the independent claims are directed to different aspects of the invention, they all define subject-matter that has been found to allow for simplified accurate measurements of properties of body fluids.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily a system for delivering a sensing device into a body,
Fig. 2 shows schematically and exemplarily a sensing device for measuring a property of a body fluid inside the body,
Fig. 3 shows schematically and exemplarily a further sensing device for measuring a property of a body fluid inside the body,
Fig. 4 shows schematically and exemplarily a system for acquiring signals from a sensing device and reference measurement data from a reference sensor of the system,
Fig. 5 shows schematically and exemplarily excitation pulses used for exciting a response of a sensing device, together with a corresponding response signal acquired from the sensing device,
Fig. 6 shows schematically and exemplarily calibration data and a corresponding calibration curve for calibrating a sensing device, and
Fig. 7 shows schematically and exemplarily supplementary calibration data.

### DETAILED DESCRIPTION OF EMBODIMENTS

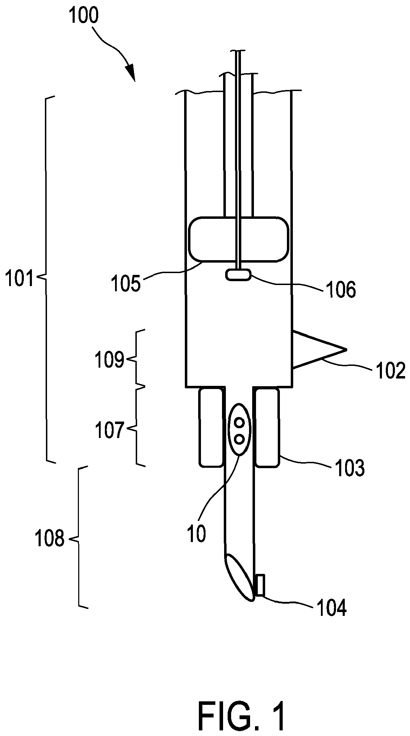

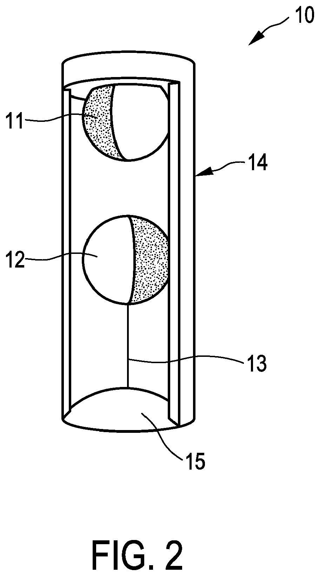

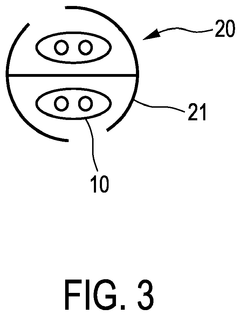

Fig. 1 shows schematically and exemplarily a system 100 for delivering a sensing device 10 into a body, wherein the sensing device 10 allows to measure a property of a body fluid inside the body. The sensing device 10 could be a magneto-mechanical resonator as schematically and exemplarily illustrated by Fig. 2, which will be described in more detail further below. According to an alternative embodiment, the sensing device deliverable by the system 100 into the body could be of the type 20, as schematically and exemplarily illustrated by Fig. 3, and as will also be described in more detail further below.

Fig. 1 corresponds to an axial cross-section through the system 100 along its longitudinal axis, wherein the longitudinal axis of the system 100 can be assumed to be an axis of approximate rotational symmetry of a casing of the system 100, at least in the part visible in Fig. 1. Fig. 1 only shows a distal region of the system 100, i.e. a region of the system 100 by which the system 100 will first approach the body when the sensing device 10 is delivered into the body. More proximal parts, such as actuating elements for the delivery mechanism realized by the system 100, for instance, are not shown.

The casing of the system 100 defines a first, proximal cylindrical portion and a second, distal cylindrical portion, wherein the first, proximal cylindrical portion has a diameter that is approximately three times as large as the diameter of the second, distal cylindrical portion. The second, distal cylindrical portion is in this case a needle. A proximal part of the second, distal cylindrical portion, i.e. of the needle, defines a distal part of a storage portion 101 in which one or more of the sensing devices 10 can be stored before their delivery. For instance, the one or more sensing devices 10 can be clamped against inner walls of the distal part of the storage portion 101. The first, proximal cylindrical portion of the system 100, although in this case it does itself not store any sensing device, is still regarded as being part of the storage portion 101 of the system, such that the actual storage position of the sensing device 10 is at a lower, distal part of the storage portion 101, while the upper, proximal part of the storage portion 101 just serves as a storage for withdrawn body fluid. Storing the sensing device 10 in a relatively narrow part of the system 100, such as the proximal, or upper, part of a distal needle used for injection as seen in Fig. 1, also has the advantage that the stored sensing device 10 will likely already be aligned correctly for an easy delivery.

The system 100 can be used to withdraw body fluid from the body, i.e. the body into which the one or more sensing devices 10 are to be delivered, into the storage portion 101. In the illustrated embodiment, this is achieved by actuating a piston 105, as will be described in more detail further below. The system 100 further comprises a reference sensor 102 that is configured to carry out a reference measurement of the property to be measured by the sensing device 10, wherein the reference measurement is carried out on body fluid withdrawn, i.e. with the system 100, from the body into the storage portion 101. After the reference measurement has been carried out, the system 100 can be used so as to deliver the one or more sensing devices 10 from the storage portion 101 into the body, typically along with some or even all of the body fluid previously withdrawn. In the illustrated embodiment, this is achieved by optionally actuating the piston 105, but at least an additional piston 106 in the form of a sub-piston, as will also be described in more detail further below.

Using the system 100, the one or more sensing devices 10 can be calibrated inside body fluid without having to actually inject them into the body. The reference sensor 102 can insofar be regarded as replacing invasive reference measurement means like catheters. The storage portion 101 could therefore also be regarded as an external calibration region, wherein "external" refers to being positioned external to the body.

Sensing characteristics of many sensing devices are often significantly affected by temperature, i.e. the temperature of a medium in which the sensing device is situated. In order to allow for an adequate calibration of the one or more sensing devices used herein, the system 100 further comprises a temperature control unit 103 configured to control a temperature of the body fluid withdrawn from the body into the storage portion 101. The temperature control unit 103 can comprise a temperature sensor for measuring a temperature of the withdrawn body fluid and a heater for heating the withdrawn body fluid, wherein the heater may be controlled to heat the withdrawn body fluid based on its temperature as measured using the temperature sensor of the temperature control unit 103. Apart from the temperature sensor of the temperature control unit 103, the system 100 comprises a further temperature sensor 104 at a distal end, wherein this further temperature sensor 104 may be used for measuring the temperature of the body fluid inside the body. Maintaining the body fluid withdrawn from the body at the temperature it has inside the body, which can be preferred, can be done more accurately with the additional temperature sensor 104. For instance, the temperature control unit 103 can receive the body fluid temperature inside the body as measured by the further temperature sensor 104 as a set point at which to hold the temperature of the withdrawn body fluid in the system 100, wherein for holding the temperature of the withdrawn body fluid at the set point a control loop involving temperature measurements of the temperature sensor of the control unit 103 itself may be followed.

The storage portion 101 in Fig. 1 could be regarded as encompassing an upper part and a lower part, wherein the upper part is larger in diameter than the lower part, and the lower part, in which the sensing device 10 is in this case stored, forms an uppermost end section of an injection needle further described below. The more proximal part of the storage portion 101, which in this case just stores withdrawn body fluid, forms a reference measurement portion 109, as it comprises the reference sensor. The lower part forms a temperature control portion 107, as, besides storing the sensing device 10, it comprises the temperature control unit 103.

The larger diameter of the reference measurement region 109 as compared to the temperature control region 107 corresponds to a larger extent in a transverse direction with respect to a delivery direction. The delivery direction corresponds in Fig. 1 to the longitudinal axis, when viewed from a proximal side to a distal side of the system 100. A transverse direction could be any direction non-parallel (and also not anti-parallel) to the delivery direction. However, in the illustrated embodiment it may particularly refer to a direction perpendicular to the delivery direction, such that the extent of the storage portion 101 in its upper part can be compared to its extent of its lower part in terms of a respective diameter of the respective cylinders defining the shapes of the two portions. In Fig. 1, the reference measurement portion 109, like the whole upper part of the storage portion 101, has a diameter that is approximately three times as large as the diameter of the temperature control portion 107, which itself corresponds to the upper part of the injection needle discussed below. Using such a relatively bulky reference measurement portion 109 and such a relatively slender injection portion allows for a gentle and accurate injection of the one or more sensing devices 10 into the body while at the same time allowing for sufficient convection of the withdrawn body fluid inside the system 100. Convection in the withdrawn body fluid can support an acclimatization of the one or more sensing devices 10 to the withdrawn body fluid, particularly its temperature, thereby allowing for a quick and accurate calibration.

As already mentioned, the reference sensor 102 is, in the illustrated embodiment, part of the reference measurement portion 109 of the system 100. It could be considered to be positioned partially on the storage portion 101 (specifically the reference measurement portion 109) and partially in the storage portion 101 (specifically the reference measurement portion 109), since a sensing part of it is to get in direct contact with at least some withdrawn body fluid in the storage portion 101, whereas another part of it is to transmit and/or conduct a corresponding measurement signal to the outside for further processing in the course of calibration.

The temperature control unit 103 is, as seen in Fig. 1, circumferentially mounted to the casing of the system 100, particularly at what could be regarded as a neck, or shoulder, region formed at a transition between the reference measurement region 109 and the temperature control region 107. This allows for an efficient temperature control of body fluid being withdrawn from the body and flowing into the storage portion 101, particularly passing by the temperature control portion 107 and into the reference measurement portion 109.

For the reference pressure sensor 102, the temperature sensor of the control unit 103 and the further temperature sensor 104, any known sensor types can be used. It will be understood that the heater of the temperature control unit 103 is preferably an electrical heater. Moreover, it will be appreciated that the sensors preferably provide sensor data are used for calibration via one or more analog-to-digital converters.

As shown in Fig. 1, the system 100 comprises, in the illustrated embodiment, an injection portion 108 as a distal part of an injection needle located distally relative to the storage portion 101. The injection portion 108 is to be inserted into a delivery region inside the body. A proximal part of the injection needle corresponds to the above described temperature control portion 107. The injection portion 108 allows to establish a fluid-conducting relationship between a delivery region inside the body and the storage portion 101 of the system 100. In other words, body fluid can be withdrawn from the delivery region through the injection portion 108 into the storage portion 101, and the sensing device 10 can be injected from the storage portion 101 through the injection portion 108 into the delivery region inside the body. In the illustrated case of a needle forming the temperature control portion 107 and the injection portion 108, the fluid-conducting relationship is established by inserting the distal tip of the needle, which is preferably sufficiently sharp or pointed to break apart skin, into the body and to the delivery region, such that body fluid from the delivery region can flow through an interior of the needle and enter the storage portion 101, particularly the reference measurement portion 109 through an opening connecting the interior of the needle 107, 108 with the reference measurement portion 109.

As still illustrated by Fig. 1, the system 100 can comprise a pressure generator in the form of a double piston formed from a main piston 105 and a sub-piston 106, wherein the sub-piston 106 is arranged so as to slide within an interior of the main piston 105 along its longitudinal, i.e. movement, axis. Both pistons are positioned in, and movable inside, the storage portion 101 of the system 100, wherein particularly the position of the main piston 105 may dictate a volume of the storage portion 101 that is fillable by withdrawn body fluid. The pressure generator thus formed is suitable to generate underpressure inside the system 100 for withdrawing the body fluid from the body into the storage portion 101, and to generate a mechanical force on the one or more sensing devices 10 for delivering the one or more sensing devices 10 from the storage portion 101 into the body.

For instance, body fluid from the body can be withdrawn after insertion of the injection portion 108 into the body, thereby establishing a fluid conducting relationship between the delivery region and the storage portion 101, by pulling at least the main piston 105 from a first position to a second position, wherein the first position is more distal relative to the second position. If this is done after insertion of the injection portion 108, an underpressure will be generated relative to the delivery region that will support a withdrawal of body fluid from the body into the storage portion 101. However, creating such an underpressure may not be necessary, since the pressure of the body fluid inside the body may be sufficient to establish a flow of body fluid from the body into the storage portion 101. Hence, it can be sufficient to position the main piston 105 in the second position before injection of the injection portion 108 into the body, or in any other position leaving a sufficiently large volume in the storage portion 101 to be filled by the withdrawn body fluid. Particularly in this latter case, a venting mechanism may be provided in a proximal region of the system 100, in order to prevent a pressure equilibration between the storage portion 101 and the delivery region inside the body, which may potentially stop the flow of body fluid.

Once a sufficient amount of body fluid has been withdrawn into the storage portion 101, thereby immersing the one or more sensing devices 10 stored in the storage portion 101, and once the one or more sensing devices 10 have, possibly after a suitable acclimatizing time, been calibrated, the one or more sensing devices 10 can be delivered into the body for carrying out measurements. This can be done by a) repositioning the piston 105 from the second position to the first position, thereby generating an overpressure decreasing the fluid-accessible volume in the interior of the system 100 such that any sensing devices 10 stored in the previously withdrawn body fluid flow back along with part of the body fluid towards the distal side of the system 100 and finally the delivery region inside the body, and/or b) moving the piston 106 in the delivery direction, particularly also through the injection portion 108 of the system 100 so as to push any left-over sensing devices 10 through the injection portion 108. For this purpose, a distal end of the piston 106, which is a piston head, may be fitted in size to the diameter of the injection needle, or generally the temperature control portion 107 and the injection portion 108, similarly as a piston head of the piston 105 may be fitted in size to the diameter of the upper, broader part of storage portion 101, comprising the reference measurement region 109. As seen in Fig. 1, the stored sensing device 10 is already aligned with the needle used for insertion, such that it is relatively easy for the piston 106 to push the sensing device 10 through the middle and into the body, i.e. as compared to a case where, for instance, the sensing device 10 would be stored in the upper broader part of the storage portion 101, in which the orientation of the sensing device 10 would be less controllable. Nevertheless, embodiments are possible in which the sensing device 10 is not stored in exactly the position shown in Fig. 1. For instance, as long as an alignment of the sensing device 10 with the needle used for injection should be maintained, a tube could extend from the needle in a proximal direction, i.e. into the broader part of the storage portion 101 referred to above as reference measurement region 107, and the sensing device 10 could be stored inside this tube. The tube would preferably have a same or similar diameter as the needle, thereby allowing to preserve the alignment of the sensing device 10. The tube may have perforations to allow withdrawn body fluid to pass by.

Fig. 2 schematically and exemplarily shows an embodiment of a sensing device in the form of a pressure sensor 10 for being introduced into the circulatory system of a human being. The pressure sensor 10 comprises a magneto-mechanical resonator with two magnetic elements 11, 12.

The magnetic element 12 is suspended from a filament 13 and is thus free to perform a rotational motion about the resonator main axis. In this embodiment the further magnetic object 11 is fixed. However, in another embodiment the further magnetic element can also be suspended from a filament and thus can be free to perform a rotational motion about the resonator main axis.

In equilibrium, the magnets 11, 12, respectively, align with anti-parallel orientation of their magnetization. An external magnetic field pulse can be used to start a resonance rotational oscillation. The attractive force exerted by the two magnets 11, 12 upon each other determines the resonance frequency of the oscillation.

The field variation generated by the oscillating magnetic element can be detected via the induced voltage in one or several detection coils of a transducer, which is configured to transduce a magnetic or electromagnetic field generated by the mechanical oscillations of the magnetic object 11 of the pressure sensor 10 into electrical response signals. The time trace of the detected signal can be Fourier-transformed to obtain the spectrum, which enables determination of the resonance frequency.

Generally, at least a part of the casing 14 of the sensor 10 will be chosen to be flexible for allowing to transduce external pressure changes into changes of the mechanical oscillation of the magnetic object 11, 12. In the illustrated embodiment, the casing 14 comprises a deflectable membrane 15. The deflection of this membrane depends on pressure exerted on the sensor 10 and changes the intersphere distance, i.e. the distance between the two magnets 11, 12. A reduction in distance results in an increase of the attractive force between the two magnets 11, 12, and thereby to an increase of the resonance frequency that can be measured for pressure determination.

Fig. 3 shows schematically and exemplarily a sensing device 20 particularly suitable for measuring a wedge pressure. The sensing device 20 comprises a substantially spherical casing, wherein Fig. 3 shows a cross-section through the device. Two sensing devices 10 of the kind described with respect to Fig. 2 above are stored in the casing, each in a respective chamber formed in the interior of the casing. The chambers are separated from each other by a separating wall lying in an equatorial plane of the sensing device 20. The casing of the sensing device 20 comprises two openings on opposite sides, wherein body fluid can enter into the interior of the device 20 through the openings. Since the openings are positioned opposite to each other and are relatively small, such that one opening is associated to each of the chambers, it is likely that, when the device 20 has reached a position in which it blocks a flow of body fluid, such as a narrowing vessel structure, one of the openings will be positioned upstream and the other of the openings will be positioned downstream, with the wall separating the two chambers in the device 20 preventing fluid flow through the device 20. Hence, at least the downstream pressure will likely always be measurable with one of the sub-devices 10 included in the device 20. This makes the sensing device 20 particularly suitable for wedge pressure measurements.

Any of the sensing devices shown in Fig. 2 and Fig. 3 can be manufactured from a material that is dissolvable in the body fluid, such as poly-lactic acid.

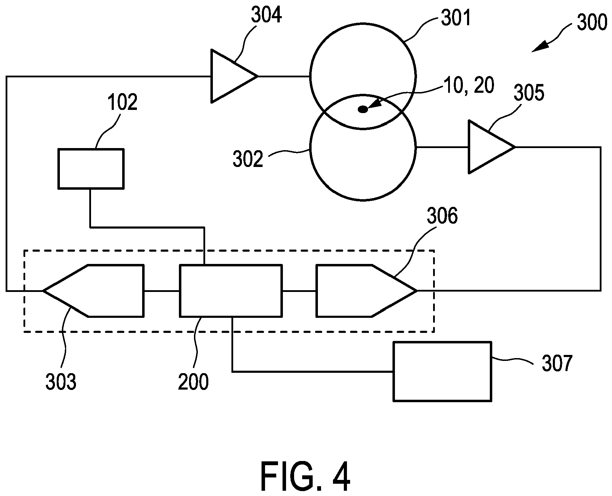

Fig. 4 shows schematically and exemplarily a system 300 that can be used to acquire signals from sensing devices 10, 20 like the ones shown in Fig. 2 and Fig. 3, particularly for calibrating the sensing devices 10, 20 and thereafter using the calibrated sensing devices 10, 20 to measure the body fluid property of interest, particularly the blood pressure of a patient. The system 300 could therefore be regarded as a signal acquisition system, a calibration system and/or a measurement system.

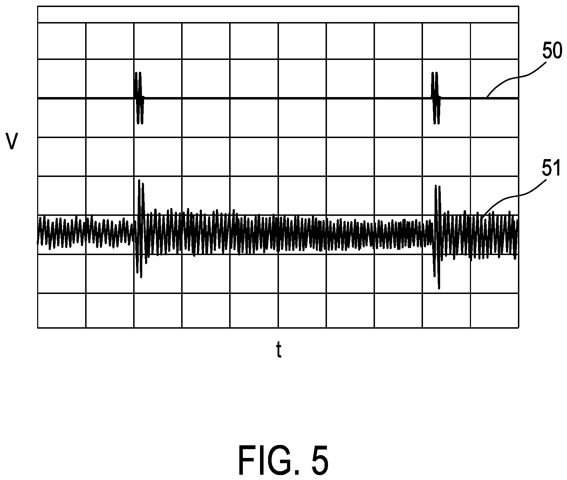

The system 300 comprises a controller 200 controlling a field generator 301 in the form of one or more transmit coils for generating a magnetic or electromagnetic excitation field for inducing mechanical oscillations of a respective magnetic object in the respective sensing device 10, 20, and a transducer 302 in the form of one or more receive coils for transducing a magnetic or electromagnetic field generated by the induced mechanical oscillations of the respective magnetic object of the respective sensing device 10, 20 into electrical response signals, wherein the controller 200, which could insofar also be regarded as an evaluation unit, determines a pressure value, or more generally a value of the property to be measured, based on the electrical response signals. The system 300 comprises in this case a digital-to-analog converter 303 and an audio amplifier 304 via which the field generator 301 is connected with the controller 200. Moreover, the system 300 includes a low noise amplifier 305 and an analog-to-digital converter 306 via which the transducer 302 is connected to the controller 200. The acquired response signals can be handed over by the controller 200 to a display 307 for displaying them. Fig. 5 shows, in terms of the upper signal trace 50, two exemplary transmitted excitation pulses generated using the transmit coils and, in terms of the lower signal trace 51, the corresponding induced voltage in the receive coils.

For calibrating one or more sensing devices 10, 20 stored in the system 100, i.e. to be delivered into the body, the system 300 may function as a calibration apparatus. For this purpose, the controller 200 can implement i) a calibration signal providing unit configured to provide a calibration signal, the calibration signal being a signal acquired from the respective sensing device 10, 20, with the sensing device 10, 20 being stored in the storage portion 101 of the system 100, after withdrawal of body fluid into the storage portion, ii) a reference measurement data providing unit configured to provide reference measurement data acquired contemporaneously with the calibration signal using the reference sensor 102, and ii) a calibration unit configured to calibrate the sensing device 10, 20 based on a) the calibration signal and b) the reference measurement data. The calibration signal may be acquired as previously described with respect to Fig. 4, wherein the reference measurement data may be received from the reference sensor 102, which can be viewed as being part of the system 300, as indicated in Fig. 4.

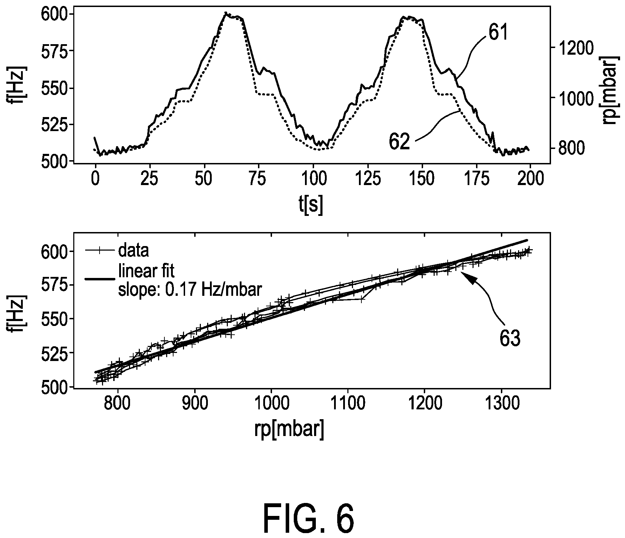

The calibration unit, which may be implemented by the controller 200 as indicated above, is configured to calibrate the sensing device 10, 20 based on a calibration signal and reference measurement data acquired over a calibration time and/or at least at a time at which the sensing device 10, 20 has thermally equilibrated with the withdrawn body fluid to a predetermined degree. A typical calibration is schematically and exemplarily illustrated by Fig. 6. Fig. 6 shows in its upper part the resonance frequency 61 determined based on the calibration signal acquired from a sensing device 10, 20, and the reference pressure 62 determined based on the reference measurement data recorded with the reference sensor 102. Their approximate alignment indicates the suitability of the sensing device 10, 20 as a pressure sensor, namely by measuring its resonance frequency and determining the pressure based thereon. In its lower part, Fig. 6 shows a calibration curve 63 based on which, for a given measured resonance frequency of the sensing device, a pressure can be determined after delivery of the sensing device into the body. In this case, the calibration curve 63 is a linear fit to calibration data collected over more than one sweep over the relevant pressure range, i.e. to data points of which each corresponds to a certain reference pressure measured by the reference sensor 102 and an associated resonance frequency measured in terms of a calibration signal acquired from the sensing device 10, 20.

A calibration as illustrated by Fig. 6 could be applied in the present case as a "base" calibration whose results are then improved by the calibration inside the system 100. While the "base" calibration would then mainly be for calibrating the pressure-dependence of the resonance frequency, the calibration inside the system 100 would mainly take into account the changes induced upon the sensing characteristics of the respective calibrated sensing device 10, 20 by it being subjected to the environment of body fluid at a certain temperature. For the calibration inside the system 100, which could also be regarded as a supplementary calibration, a one-point or two-point calibration can be sufficient. For a one-point calibration, the difference between a) the pressure value measured with the sensing device 10, 20 inside the system 100 at a given measured reference pressure and b) the pressure value that would be expected to be measured by the sensing device 10, 20 for the given measured reference pressure according to the "base-calibration" is determined, wherein this difference is added as an offset to the "base-calibrated" pressure values. In a two-point calibration, also a gain, or more generally a predetermined parameter of the relation between resonance frequency of the sensing device 10, 20 and the reference, or "true", pressure, is adjusted for in the pressure values measured by the device 10, 20. Although not necessary to arrive at already satisfactory results, a yet more accurate calibration could be achieved by taking into account all non-linearities and hysteresis effects.

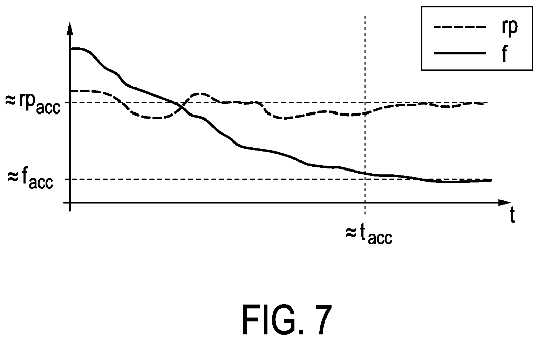

Fig. 7 shows schematically and exemplarily how the resonance frequency (f) of a respective pressure sensing device 10, 20 inside the system 100 changes as the sensing device 10, 20 acclimatizes to the body fluid withdrawn into the system 100. With its resonance frequency, also the pressure value obtained with the sensing device 10, 20 would change if the "base-calibration" were the only calibration carried out, even though the "true" pressure might essentially not change - as indicated by an approximately constant reference pressure value (rp) measured by the reference pressure sensor 102. The values f_{acc} and rp_{acc} measured at some acclimatization time t_{acc} after beginning of fluid withdrawal, for instance, could be used for the supplementary one-point calibration indicated above.

The calibration unit can also be configured to calibrate a collision behavior of the sensing device 10, 20 based on mechanical collisions between the sensing device 10, 20 and its surroundings detected in the calibration signal. For instance, sudden changes in the signal received from the sensing device 10, 20 due to deformations arising from being pushed accidentally too hard by the piston 106, or from collisions of the sensing device 10, 20 with inner walls of the system 100 or potentially further delivered sensing devices can be detected, and a collision behavior of the sensing device can be determined based thereon. If sudden changes are again detected during measurement inside the body, which may arise from collisions with tissue structures, the determined collision behavior can be used to correct measurement signal.

An exemplary workflow for preparing a measurement of a property of a body fluid inside the body, which amounts to a preferred use of the system 100, could be summarized by the following steps: Firstly, one or more sensing devices 10, 20 are stored in the storage portion 101 of the system 100. This step does not necessarily need to be taken by a user of the system 100, but could also be taken beforehand, such as during production of the system 100, or its preparation for use. With the one or more sensing devices 10, 20 stored in the system 100, body fluid is withdrawn from the body into the system 100, particularly the storage portion 101. As described above, the system 100 can comprise a piston arrangement for this purpose, in which case the user may actuate, such as by pulling, the piston arrangement for withdrawing the body fluid. While or after the body fluid is withdrawn, the reference sensor 102 is used to carry out a reference measurement of the property to be measured by the one or more sensing devices 10, 20. Hence, the reference measurement is carried out on body fluid withdrawn by the system 100 from the body into the storage portion 101. Carrying out the reference measurement may refer to a recording of measurement data collected by the reference sensor 102. Furthermore, while the reference measurement is carried out, the stored one or more sensing devices 10, 20 are used to carry out a calibration measurement of the property to be measured by the one or more sensing devices 10, 20. Thus, also the calibration measurement is carried out on the body fluid withdrawn by the system 100 from the body into the storage portion 101. Preferably, the reference measurement and the calibration measurement are carried out contemporaneously, such that pairs of a) reference measurement data and b) calibration measurement data can be formed, wherein each pair corresponds to a given time during the calibration process. The calibration measurement corresponds to an acquisition of a calibration signal from the one or more sensing devices 10, 20, as already described further above. The one or more sensing devices 10, 20 are calibrated based on the reference measurement data and the calibration signal. Hence, for calibrating the one or more sensing devices 10, 20, a calibration signal being a signal acquired from the one or more sensing devices 10, 20 stored in the storage portion 101 of the system 100 after withdrawal of body fluid into the storage portion 101 is provided, reference measurement data acquired contemporaneously with the calibration signal using the reference sensor 102 are provided, and the one or more sensing devices 10, 20 are calibrated based on the calibration signal and the reference measurement data. The calibrated sensing devices 10, 20 are ready for measurement and can therefore be delivered from the storage portion 101 into the body. For instance, as described above, the piston arrangement already used for withdrawing the body fluid from the body can be used for this purpose again.

The calibration *per se,* i.e. the calibration process carried out based on the reference measurement data from the reference sensor 102 and the calibration signal from the one or more sensing devices 10, 20, can be automatized. For this purpose, a computer program for calibrating a sensing device 10, 20 allowing to a) measure a property of a body fluid inside the body and to b) be delivered into the body using the system described above is provided. The program comprises instructions causing the above described calibration apparatus, possibly implemented by the controller 200, to carry out the above described calibration method. In more sophisticated applications, in fact, also the whole workflow indicated above, possibly with the exception of the storing step, could be automatized, wherein a computer program for controlling a corresponding apparatus may be provided. Also any measurements following the calibration and subsequent delivery of the sensing devices 10, 20 can be automatized and a corresponding computer program may be provided, wherein optionally this computer program could be the above-mentioned computer program for calibration, such that the calibration and also the measurement would be controlled by the controller 200.

Although the above described embodiments mainly related to uses of the presented system in combination with sensing devices in the form of magneto-mechanical oscillators, also known as micro-mechanical resonators, particularly for measurements of pulmonary artery pressure and wedge pressure, the system can also be used for delivering other sensing devices into the body, possibly for measuring other body fluid properties, while allowing for a previous calibration of the sensing devices.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like carrying out a reference measurement, acquiring calibration signals, providing corresponding reference measurement data and calibration signals, the calibration itself, *et cetera,* performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for delivering a sensing device into a body that allows to measure at least one property of a body fluid. The system comprises a storage portion for storing the sensing device before delivery, the system allowing to withdraw body fluid from the body into the storage portion, and a reference sensor configured to carry out a reference measurement of the property to be measured, the reference measurement being carried out on body fluid withdrawn from the body into the storage portion. After the reference measurement has been carried out, the sensing device can be delivered from the storage portion into the body. The presented system allows to simplify accurate measurements of body fluids.

## Claims

1. A system (100) for delivering a sensing device (10; 20) into a body, wherein the sensing device (10; 20) allows to measure at least one property of a body fluid inside the body, wherein the system (100) comprises:
- a storage portion (101) for storing the sensing device (10; 20) before delivery, wherein the system (100) allows to withdraw body fluid from the body into the storage portion (101),
- a reference sensor (102) configured to carry out a reference measurement of the property to be measured by the sensing device (10; 20), wherein the reference measurement is carried out on body fluid withdrawn from the body into the storage portion (101),
wherein the system (100) allows to deliver the sensing device (10; 20) from the storage portion (101) into the body after the reference measurement has been carried out.

2. The system (100) as defined in claim 1, wherein the system (100) further comprises a temperature control unit (103) configured to control a temperature of the body fluid withdrawn from the body into the storage portion (101).

3. The system (100) as defined in claim 2, wherein the storage portion (101) comprises a temperature control portion (107) comprising the temperature control unit (103).

4. The system (100) as defined in claim 3, wherein the storage portion (101) is constructed so as to store the sensing device (10; 20) in the temperature control portion (107).

5. The system (100) as defined in any of the preceding claims, wherein the system (100) comprises an injection portion (108) to be inserted into a delivery region inside the body, wherein body fluid can be withdrawn from the delivery region through the injection portion (108) into the storage portion (101), and the sensing device (10; 20) can be injected from the storage portion (101) through the injection portion (108) into the delivery region inside the body.

6. The system (100) as defined in claim 5, wherein the storage portion (101) comprises a reference measurement portion (109) comprising the reference sensor (102), the reference measurement portion (109) having a larger extent than the injection portion (108) in a transverse direction with respect to a delivery direction.

7. The system (100) as defined in claim 5 or 6, wherein, for withdrawing the body fluid from the body into the storage portion (101), the system (100) comprises a pressure generator (105, 106) for generating underpressure inside the system (100) relative to the delivery region if the injection portion (108) is inserted with its distal part into the delivery region.

8. The system (100) as defined in claim 7, wherein the pressure generator (105, 106) further allows to generate a mechanical force on the sensing device (10; 20) for delivering the sensing device (10; 20) from the storage portion (101) into the body.

9. The system (100) as defined in claims 1 to 6 and optionally claims 7 or 8, wherein the system (100) extends along a longitudinal axis corresponding to the delivery direction, wherein the storage portion (101) forms a first, proximal portion of the system (100) in the delivery direction, and the injection portion (108) forms a second, distal portion of the system (100) in the delivery direction, wherein the temperature control portion (107) is a distal section of the storage portion (101) preceding the injection portion (108) in the delivery direction and having the same extent as the injection portion (108) in the transverse direction with respect to the delivery direction, and wherein the reference measurement portion (109) is a section of the storage portion (101) preceding the temperature control portion (107) in the delivery direction.

10. A calibration apparatus (200) for calibrating a sensing device (10; 20) allowing to measure at least one property of a body fluid inside the body and to be delivered into the body by the system (100) as defined in any of claims 1 to 8, the apparatus (200) comprising:
- a calibration signal providing unit configured to provide a calibration signal, the calibration signal being a signal acquired from the sensing device (10; 20) with the sensing device (10; 20) being stored in the storage portion (101) of the delivery (100) device after withdrawal of body fluid into the storage portion (101),
- a reference measurement data providing unit configured to provide reference measurement data using the reference sensor (102), and
- a calibration unit configured to calibrate the sensing device (10; 20) based on a) the calibration signal and b) the reference measurement data.

11. The calibration apparatus (200) as defined in claim 10, wherein the calibration unit is configured to calibrate the sensing device (10; 20) based on a calibration signal and reference measurement data acquired over a calibration time and/or at least at a time at which the sensing device (10; 20) has thermally equilibrated with the withdrawn body fluid to a predetermined degree.

12. The calibration apparatus (200) as defined in any of claims 10 and 11, wherein the calibration unit is configured to calibrate a collision behavior of the sensing device (10; 20) based on mechanical collisions between the sensing device (10; 20) and its surroundings detected in the calibration signal.

13. A calibration method for calibrating a sensing device allowing to measure at least one property of a body fluid inside the body and to be delivered into the body by the system (100) as defined in any of claims 1 to 9, the method including:
- providing a calibration signal, the calibration signal being a signal acquired from the sensing device with the sensing device being stored in the storage portion (101) of the system (100) after withdrawal of body fluid into the storage portion (101),
- providing reference measurement data acquired using the reference sensor (102), and
- calibrating the sensing device (10; 20) based on a) the calibration signal and b) the reference measurement data.

14. A method for using the system as defined in any of claims 1 to 9, wherein the method includes:
- storing the sensing device (10; 20) in the storage portion (101),
- withdrawing body fluid from the body into the storage portion (101),
- carrying out, using the reference sensor (102), a reference measurement of the property to be measured by the sensing device (10; 20), wherein the reference measurement is carried out on body fluid withdrawn by the system (100) from the body into the storage portion (101),
- carrying out, using the stored sensing device (10; 20), a calibration measurement of the property to be measured by the sensing device (10; 20), wherein also the calibration measurement is carried out on the body fluid withdrawn by the system (100) from the body into the storage portion (101),
- calibrating the sensing device (10; 20) based on the reference measurement and the calibration measurement,
- delivering the sensing device (10; 20) from the storage portion (101) into the body.

15. A computer program for calibrating a sensing device (10; 20) allowing to measure a property of a body fluid inside the body, delivered into the body using the system (100) as defined in any of claims 1 to 9, the program comprising instructions causing the calibration apparatus (200) as defined in any of claim 10 to 12 to carry out the calibration method as defined in claim 13.
